# EUROPEAN PATENT APPLICATION

(11) **EP 4 830 769 A2**
(43) Date of publication of application: **28.10.2026**
(21) Application number: 26205013.1
(22) Date of filing: 02.08.2024
(51) Int. Cl.: A61N 1/375

(54) **BIOSTIMULATOR HAVING FIXATION GUIDE**

(30) Priority: 03.08.2023 US 202363530670 P; 01.08.2024 US 202418792438
(62) Document number(s) of the earlier application(s) in accordance with Art. 76 EPC: 24192590.8 / 4 501 395
(71) Applicant: Pacesetter, Inc., Sylmar, CA 91342 (US)
(72) Inventor: ARNAR, Bernhard, Sylmar, Ca 91342 (US); JACKSON, Aaron, Sylmar, CA 91342 (US)
(74) Representative: Barker Brettell Sweden AB

(57) **Abstract**

A biostimulator (100) includes a housing (110) having an electronics compartment (302) containing pacing circuitry. A fixation guide (120) is mounted on the housing (110) and includes a guide passage (312). A fixation element (106) is movable through the guide passage (312) from an undeployed state to a deployed state. In the undeployed state, a fixation tip (604) of the fixation element (106) is within the guide passage (312). In the deployed state, the fixation tip (604) extends out of the guide passage (312). A biostimulator system (200) includes the biostimulator (100) mounted on a biostimulator transport system (202) to deliver the biostimulator (100) to, and extend the fixation tip (604) into, a target anatomy. Other embodiments are also described and claimed.

## Description

### TECHNICAL FIELD

The present disclosure relates to biostimulators and related biostimulator systems. More specifically, the present disclosure relates to leadless biostimulators and related systems useful for septal pacing.

### BACKGROUND INFORMATION

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricle apex. More particularly, a pacing site for LBB pacing is typically below the His-bundle, on the interventricular septal wall. To achieve optimal results, the pacing site for physiological pacing at the LBB can be high on the interventricular septal wall, in the region close to the tricuspid valve and pulmonary artery outflow track. Furthermore, the pacing site may be at a depth of up to 1.5 cm within the septal wall.

### SUMMARY

Existing leadless pacemakers may not fit, or may interfere with heart structures, when placed at the optimal pacing site for left bundle branch (LBB) pacing. More particularly, existing leadless pacemakers have bodies that are long and rigid and, when implanted at the interventricular septal wall, could extend into contact with the cardiac tissue of a ventricular free wall or the tricuspid valve during contraction of the heart. The long and rigid body of existing leadless pacemakers could also become tangled within chordae tendinae. Furthermore, a proximal end of the existing leadless pacemakers may flail within the heart chamber as the heart beats, causing cyclical contact with adjacent heart structures. Such contact could interfere with heart function. Thus, there is a need for a leadless biostimulator that can be engaged to the interventricular septal wall to pace the LBB without interfering with adjacent structures of the heart.

The present invention is defined in the independent claim. Further embodiments of the invention are defined in the dependent claims.

A biostimulator is described. In an embodiment, the biostimulator includes a housing, a fixation guide, and a fixation element. The housing has an electronics compartment containing pacing circuitry. The fixation guide is mounted on the housing and includes a guide passage. The fixation element is movable through the guide passage from an undeployed state to a deployed state. In the undeployed state, a fixation tip of the fixation element is within the guide passage. In the deployed state, the fixation tip extends out of the guide passage. The fixation element can transition from the undeployed state to the deployed state to anchor the biostimulator in a target anatomy.

A biostimulator system is described. In an embodiment, the biostimulator system includes a biostimulator transport system. The biostimulator can be mounted on the biostimulator transport system to carry the biostimulator to or from the target anatomy.

The above summary does not include an exhaustive list of all aspects of the present invention. It is contemplated that the invention includes all devices, systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a perspective view of a biostimulator having a fixation guide, in accordance with an embodiment.
FIG. 4 is a side view of a biostimulator in an undeployed state, in accordance with an embodiment.
FIG. 5 is a side view of a biostimulator in an undeployed state, in accordance with an embodiment.
FIG. 6 is a cross-section view of a biostimulator in an undeployed state, in accordance with an embodiment.
FIG. 7 is a perspective view of a biostimulator having an actuated fixation guide, in accordance with an embodiment.
FIG. 8 is a perspective view of a biostimulator in a deployed state, in accordance with an embodiment.
FIG. 9 is a perspective view of a biostimulator in a deployed state, in accordance with an embodiment.
FIG. 10 is a side view of a biostimulator having a fixation guide, in accordance with an embodiment.
FIG. 11 is a side view of a biostimulator in an undeployed state, in accordance with an embodiment.
FIG. 12 is a front view of a biostimulator in an undeployed state, in accordance with an embodiment.
FIG. 13 is a perspective view of a biostimulator having an actuated fixation guide, in accordance with an embodiment.
FIG. 14 is a perspective view of a biostimulator in a deployed state, in accordance with an embodiment.
FIG. 15 is a perspective view of a biostimulator in a deployed state, in accordance with an embodiment.
FIG. 16 is a side view of a biostimulator in an undeployed state, in accordance with an embodiment.
FIG. 17 is a front view of a biostimulator in an undeployed state, in accordance with an embodiment.
FIG. 18 is a cross-sectional view of a biostimulator in an undeployed state, in accordance with an embodiment.
FIG. 19 is a cross-sectional view of a biostimulator in a deployed state, in accordance with an embodiment.
FIG. 20 is a cross-sectional view of a biostimulator in an undeployed state, in accordance with an embodiment.
FIG. 21 is a flowchart of a method of implanting a biostimulator for pacing.

### DETAILED DESCRIPTION

Embodiments describe a biostimulator and a biostimulator system for pacing, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a fixation guide to guide a fixation element into a target anatomy, such as an interventricular septal wall. The fixation element can move through a guide passage of the fixation guide from a position in which portion of the fixation element is parallel to a housing of the biostimulator to a position in which the portion is orthogonal to the housing. More particularly, the fixation element can be guided to a perpendicular, or near perpendicular, position relative to the housing. During implantation, the fixation element can therefore penetrate a surface of the target anatomy, e.g., the septal wall, and the housing can sit flat against the surface rather than protrude outward. Accordingly, when the fixation element is anchored in a septal wall of a heart, the housing can be located along the septal wall, e.g., in the ventricular apex, without interfering with a heart valve or a free heart wall opposite to the septal wall. The biostimulator therefore fits well within the limited space of a target heart chamber, and provides long-term implant stability and reduced risk of tissue trauma.

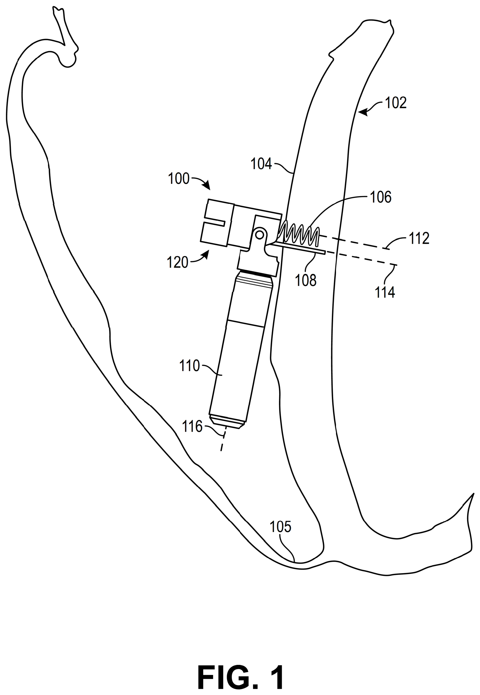

Referring to FIG. 1, a diagrammatic cross section of a patient heart illustrating an example implantation of a biostimulator in a target anatomy is shown in accordance with an embodiment. The diagram shows a biostimulator 100 attached to a patient heart 102 with a body of the biostimulator positioned toward a ventricular apex. A leadless biostimulator system, e.g., a cardiac pacing system, can include the biostimulator 100. The biostimulator 100 can be implanted in the patient heart 102, and can be leadless (and thus, may be a leadless cardiac pacemaker). The biostimulator 100 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to an interventricular septum, and one or more elements, such as a fixation element 106 and/or a pacing electrode 108, can pierce the interventricular septal wall 104 of the septum to engage and anchor the biostimulator 100 to the tissue. The biostimulator 100 can have a housing 110, which can be directed toward the ventricular apex 105, or otherwise positioned oblique or orthogonal to the fixation element 106 and/or the pacing electrode 108, as described below.

The fixation element 106 can have a fixation axis 112. The pacing electrode 108 can have a pacing axis 114. The axes 112, 114 can be directed toward, e.g., normal to, the septal wall 104 when the fixation element 106 is affixed to the septal wall and the pacing electrode 108 is inserted into the target tissue. By contrast, the housing 110 can have a housing axis 116, which is directed away from, e.g., oblique to, the septal wall 104 when the pacing electrode 108 is affixed to the septal wall. For example, the housing axis 116 can be directed toward an apex wall of the ventricular apex 105 and the housing 110 may be located therein.

When the fixation element 106 and the pacing electrode 108 are affixed to the interventricular septal wall 104, and the housing 110 is located at the ventricular apex 105, the fixation axis 112 and the pacing axis 114 can extend in a different direction than the housing axis 116. For example, the axes 112, 114 can extend in a direction that is transverse or oblique to a direction of the housing axis 116. The non-coaxial and/or non-parallel relationship of the fixation axis 112 and/or the pacing axis 114 to the housing axis 116 may be provided by a fixation guide 120 of the biostimulator 100. For example, as described below, the fixation guide 120 can include a hinge mechanism to rotate the fixation element 106 and/or pacing electrode 108 relative to the housing 110, or the fixation element 106 and/or pacing electrode 108 can be advanced through a guide passage to change paths and orientation relative to the housing 110.

When the biostimulator 100 is delivered to and screwed into the septum of the heart 102, the pacing electrode 108 may be positioned for deep septal pacing at a target anatomy, such as bundle branch in the septum. For example, an active electrode of the pacing electrode 108 can be positioned at a left bundle branch in the septum. The biostimulator 100 may deliver pacing impulses through the pacing electrode 108 to the target anatomy. Accordingly, the pacing electrode 108 can be located to effectively probe and pace the target anatomy, while the housing 110 can be placed in a safe and non-obstructive location within the heart chamber.

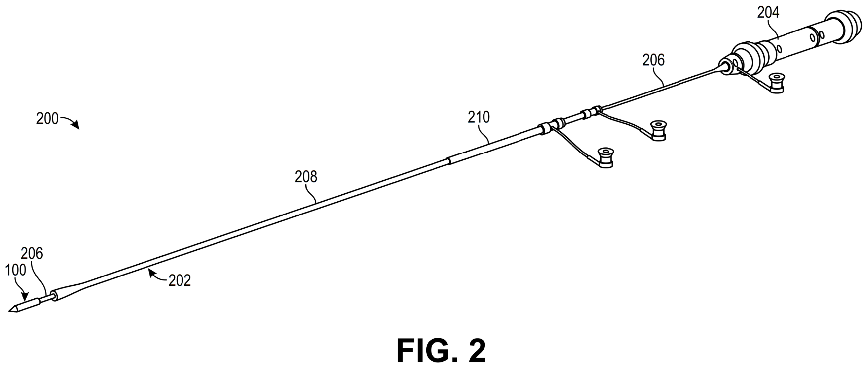

Referring to FIG. 2, a perspective view of a biostimulator system is shown in accordance with an embodiment. Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems, or biostimulator transport systems.

A biostimulator system 200 can include a biostimulator transport system 202. The biostimulator 100 can be attached, connected to, or otherwise mounted on the biostimulator transport system 202. For example, the biostimulator 100 can be mounted on a distal end of a catheter of the biostimulator transport system 202. The biostimulator 100 is thereby advanced intravenously into or out of the heart 102.

The biostimulator transport system 202 can include a handle 204 to control movement and operations of the transport system from outside of a patient anatomy. One or more elongated members extend distally from the handle 204. For example, a support member 206 can extend distally from the handle 204. The support member 206 can extend to a distal end of the transport system 202. In an embodiment, the biostimulator 100 is mounted on the biostimulator transport system 202, e.g., at a distal end of the support member 206.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator 100 during transport to the target anatomy and rotation of the biostimulator 100 during implantation of the biostimulator at the target anatomy. Accordingly, the biostimulator transport system 202 can incorporate features to retain and rotate the biostimulator 100.

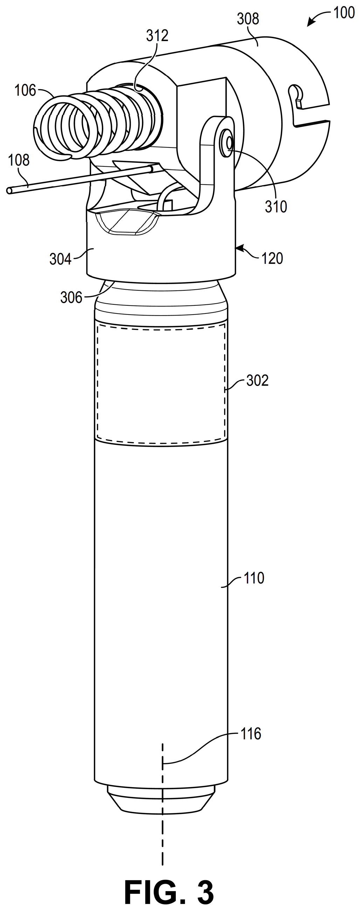

Referring to FIG. 3, a perspective view of a biostimulator having a fixation guide is shown in accordance with an embodiment. The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within a housing 110 of the biostimulator 100 for pacing the cardiac chamber upon receiving a triggering signal from at least one other device within the body. The two or more electrodes of the biostimulator 100 can include the pacing electrode 108 that acts as an active electrode and/or a portion of the housing 110 that acts as an active electrode. The electrodes can deliver pacing pulses to target anatomies, such as bundle branches within the septum of the heart 102, to perform pacing, e.g., deep septal pacing, and optionally, can sense electrical activity from the muscle. The electrodes may also communicate bidirectionally with at least one other device within or outside the body.

The housing 110 has a longitudinal axis, e.g., the housing axis 116. The housing 110 can contain a primary battery to provide power for pacing, sensing, and communication, which may include, for example, bidirectional communication. The housing 110 can optionally contain an electronics compartment 302 (shown by hidden lines) to hold circuitry adapted for different functionality. For example, the electronics compartment 302 can contain pacing circuitry for sensing cardiac activity from the electrodes, for receiving information from at least one other device via the electrodes, for generating pacing pulses for delivery to tissue via the pacing electrode 108, or other circuitry. Accordingly, the pacing circuitry can be electrically connected to the pacing electrode 108. The electronics compartment 302 may contain circuits for transmitting information to at least one other device via the electrodes and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

The biostimulator 100 can include components to fix the biostimulator 100 to an intracardial implant site, e.g., at the septal wall. More particularly, the biostimulator 100 may include one or more engaging mechanisms, such as the fixation element 106. The fixation element 106 can be an active fixation mechanism. For example, the fixation element 106 can include a fixation helix extending helically about the fixation axis 112. More particularly, the fixation helix can include a screw or helical member that screws into the target tissue. The fixation element 106 can be connected to the housing 110, e.g., via the fixation guide 120. Accordingly, when the fixation element 106 is implanted into a septal wall, the fixation guide 120 can hold the housing 110 to the fixation element 106 to anchor the housing against the septal wall.

The biostimulator 100 can include a header assembly mounted on the housing 110. The header assembly can include the fixation guide 120. Accordingly, the fixation guide 120 can be mounted on the housing 110. For example, the fixation guide 120 can include a distal guide portion 304 mounted on a proximal housing end 306 of the housing 110. The fixation guide 120 can also include a proximal guide portion 308. In an embodiment, as described further below, the fixation guide 120 can include a hinge 310 movably connecting the distal guide portion 304 to the proximal guide portion 308. Accordingly, when the hinge 310 is actuated, the fixation element 106 and the pacing electrode 108, which may be connected to the proximal guide portion 308, can move relative to the housing 110, which may be connected to the distal guide portion 304.

The proximal guide portion 308, which may be movable relative to the distal guide portion 304 and the housing 110, can support and guide one or more of the fixation element 106 or the pacing electrode 108.

In an embodiment, the proximal guide portion 308 includes a guide passage 312. The guide passage 312 can mechanically support and direct the fixation element 106 and/or the pacing electrode 108 in a radially outward direction relative to the housing axis 116 of the housing 110. Accordingly, an outer surface of the housing 110 may rest against or appose the septal wall 104 while the fixation element 106 and/or the pacing electrode 108 extend obliquely or orthogonally into the septal wall tissue.

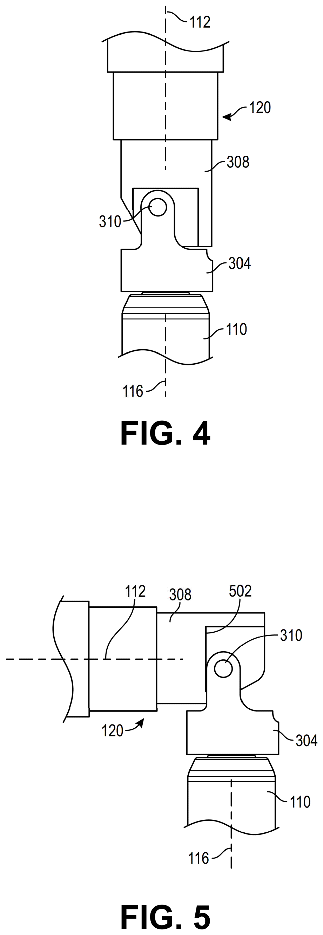

Referring to FIG. 4, a side view of a biostimulator in an undeployed state is shown in accordance with an embodiment. The hinge mechanism between the fixation element 106 and the leadless pacemaker body can move the proximal guide portion 308 relative to the housing 110 between an aligned substate and a misaligned substate. The fixation element 106 is hidden in FIG. 4, however, it is understood that the fixation element 106 extends along, e.g., helically around, the fixation axis 112. The aligned substate can be a substate of the undeployed state of the biostimulator 100. More particularly, in the undeployed state and, thus, in the aligned substate, the fixation element 106 can be housed within the proximal guide portion 308. The housed fixation element 106 can be in alignment with the housing 110, and the proximal guide portion 308 can be in alignment with the distal guide portion 304. For example, the fixation axis 112 of the fixation element 106 can be parallel to the housing axis 116 of the housing 110 when the biostimulator 100 is in the undeployed state and the hinge 310 is in the aligned substate.

Referring to FIG. 5, a side view of a biostimulator in an undeployed state is shown in accordance with an embodiment. The undeployed state of the biostimulator 100 may also include a misaligned substate. In the misaligned substate, the hinge 310 is actuated such that the proximal guide portion 308 moves into misalignment with the distal guide portion 304. When misaligned with the distal guide portion 304, the fixation axis 112 that extends through the proximal guide portion 308 may be misaligned with the housing axis 116. For example, the fixation axis 112 of the fixation element 106 can be orthogonal to the housing axis 116 of the housing 110.

The hinge mechanism can include a feature to limit movement of the proximal guide portion 308 relative to the distal guide portion 304. For example, a ledge, a pin, or another stop 502 can be incorporated into the proximal guide portion 308. The hinge 310 can have a hinge axis extending through the hinge, transverse to the fixation guide 120. The stop 502 can contact a wall of the distal guide portion 304 to limit rotation of the proximal guide portion 308 about the hinge axis.

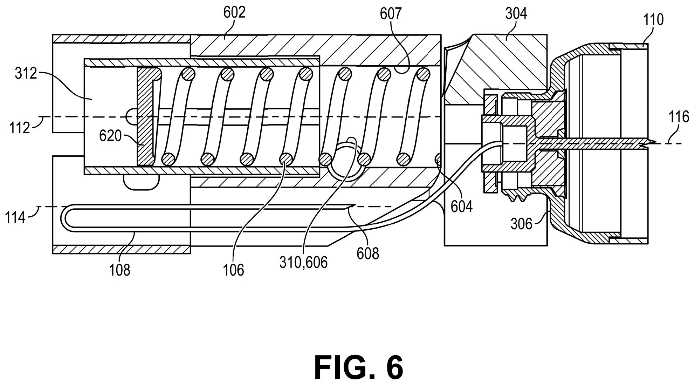

Referring to FIG. 6, a cross-sectional view of a biostimulator in an undeployed state is shown in accordance with an embodiment. The cross-section shows the hinge mechanism in the aligned substate when the biostimulator 100 is in the undeployed state. The guide passage 312 extends longitudinally through a guide body 602 of fixation guide 120. For example, the guide passage 312 can be a cylindrical bore extending from a distal end of the of the fixation guide 120 to a proximal end of the fixation guide 120. The fixation element 106 may be located within the guide passage 312. For example, the fixation element 106 can extend helically through the guide passage 312 to the fixation tip 604.

In the undeployed state, a fixation tip 604 of the fixation element 106 can be within the guide passage 312. The fixation element 106 may be movable within the guide passage 312. For example, as described below, the fixation element 106 can be moved longitudinally within the guide passage 312 to extend the fixation tip 604 from guide passage 312. In an embodiment, movement of the fixation element 106 is facilitated by a protrusion 606 within the guide body 602. The fixation guide 120 can include an inner guide wall 607 surrounding the guide passage 312, and the protrusion 606 can extend inward from the inner guide wall 607 into the guide passage 312. In an embodiment, the protrusion 606 is the hinge 310, e.g., a cylindrical rod, extending laterally through the guide passage 312.

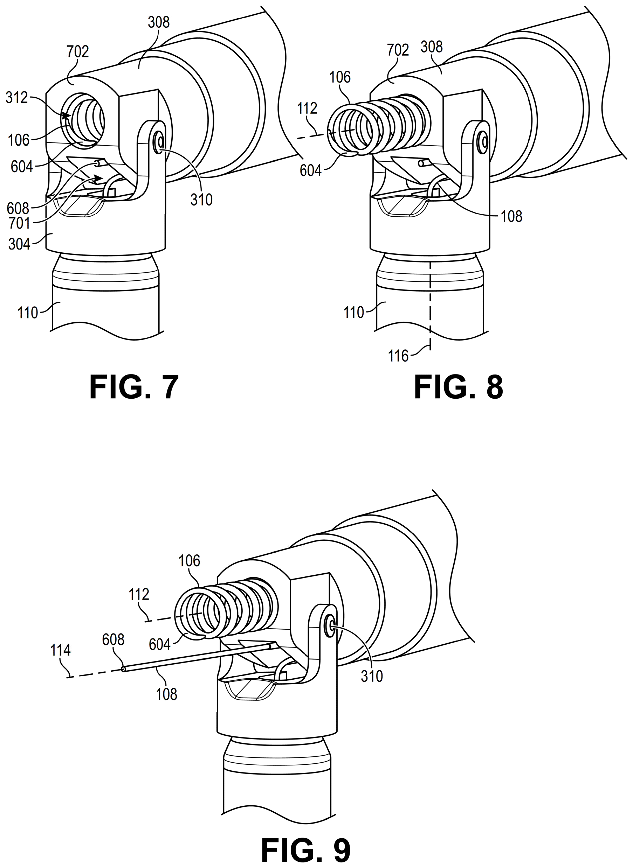

The protrusion 606 can interact with the fixation element 106 to control movement of the fixation element 106. The protrusion 606 can engage the fixation element 106. For example, the protrusion 606 can extend into the guide passage 312 between several turns of the helical fixation element 106. To advance or retract the fixation element 106, the fixation element 106 can be rotated or spun to cause the turns of the helix to thread along the protrusion 606. More particularly, the protrusion 606 can act as a mechanical thread over which the helix turns slide to result in an axial movement of the helical fixation element 106 relative to the guide body 602. The fixation element 106 can be rotated to advance the fixation tip 604 out of the guide passage 312. Thus, the fixation element 106 can be movable through the guide passage 312 from the undeployed state (e.g., FIG. 7) to the deployed state (e.g., FIGS. 8-9).

The protrusion 606 can interact with a stop tab 620 to control movement of the fixation element 106. The stop tab 620 can be a tab, e.g., a circular plate, located within the guide passage 312. The stop tab 620 can connect to and/or be fixed relative to a distal end of the fixation element 106. For example, the distal end of the fixation element 106 can be welded to the stop tab 620. Accordingly, the fixation element 106 and the stop tab 620 can be united and movement of the fixation element 106 within the guide passage 312 can be accompanied by corresponding movement of the stop tab 620. When the fixation element 106 screws forward relative to the protrusion 606, the stop tab 620 can slide forward. In an embodiment, the stop tab 620 limits movement of the fixation element 106. For example, when the stop tab 620 advances into contact with the protrusion 606, further advancement of the stop tab 620 can be resisted by the protrusion 606. The stop tab 620 accordingly retains the fixation element 106 and resists further advancement of the fixation element 106. Thus, the fixation element 106 can be retained at a forwardmost location by the stop tab 620 to avoid loss of the fixation element 106 into the target anatomy.

As described above, the fixation axis 112 can be parallel to the housing axis 116 in the undeployed state. In the aligned substate, the fixation axis 112 can be parallel, however, the fixation axis 112 may be coaxial or non-coaxial with the housing axis 116. More particularly, as shown in FIG. 6, the fixation axis 112 may not be coaxial with the housing axis 116 even though the proximal guide portion 308 is considered to be aligned with the distal guide portion 304 and the housing 110.

The pacing electrode 108 can be electrically coupled to the pacing circuitry within the housing 110. The pacing electrode 108 can extend proximally from the housing 110, e.g., through an electrical feedthrough, and extend into the fixation guide 120. In an embodiment, the pacing electrode 108 loops back distally within the fixation guide 120 and extends distally along the pacing axis 114 to a pacing tip 608. Accordingly, in the undeployed state, when the fixation guide 120 is in the aligned substate, the pacing electrode 108 can extend in a distal direction to the pacing tip 608. Similarly, the fixation element 106 can extend distally through the guide passage 312 to the fixation tip 604 in the distal direction. By contrast, the housing 110 can extend proximally in a proximal direction from a distal housing end to the proximal housing end 306 on which the distal guide portion 304 is mounted. Accordingly, the pacing electrode 108 and/or the fixation element 106 can extend in the distal direction to their respective tips, in a direction opposite to the direction that the housing 110 extends to the proximal housing end 306.

In the undeployed state, the pacing axis 114 can be parallel to the housing axis 116 of the housing 110. For example, the pacing axis 114 may be parallel to the fixation axis 112, and both axes may be parallel to the housing axis 116 in the aligned substate. The pacing axis 114 can be radially offset from the fixation axis 112. More particularly, the pacing electrode 108 can extend through the fixation guide 120 outside of the guide passage 312. For example, the fixation guide 120 can include a separate side channel radially offset from the guide passage 312, and the pacing electrode 108 can be housed and directed through the side channel.

Referring to FIG. 7, a perspective view of a biostimulator having an actuated fixation guide is shown in accordance with an embodiment. In the misaligned substate, the fixation element 106 and the fixation tip 604 can remain within the guide passage 312. For example, when the fixation guide 120 is actuated at the hinge 310 to move the proximal guide portion 308 relative to the distal guide portion 304, the fixation element 106 can remain in place within the guide passage 312. Similarly, the pacing tip 608 can remain within a side channel 701 of the proximal guide portion 308. As described below, the hinge mechanism can be actuated to lay the housing 110 along the septal wall 104, while simultaneously pressing a distal face 702 of the proximal portion against the septal wall 104 tissue.

Referring to FIG. 8, a perspective view of a biostimulator in a deployed state is shown in accordance with an embodiment. When the distal face 702 of the proximal guide portion 308 is pressed against the septal tissue, the fixation element 106 may be advanced to secure the biostimulator 100 to the septal wall 104. As described above, the fixation element 106 can be advanced by rotating the fixation element 106 relative to the proximal guide portion 308. Such rotation threads the fixation element 106 along the protrusion 606 and causes the fixation tip 604 to move out of the guide passage 312. For example, the fixation tip 604 can penetrate the tissue and the fixation helix can screw into the target tissue to secure the biostimulator 100 to the septal wall 104. Accordingly, in the deployed state, the fixation tip 604 of the biostimulator 100 can extend out of the guide passage 312.

In the deployed state, when the fixation tip 604 extends out of the guide passage 312, the fixation axis 112 can be oblique and/or orthogonal to the housing axis 116. Such obliqueness and/or orthogonality allows the fixation element 106 to be directed into the septal tissue along which the housing 110 lies parallel against.

Referring to FIG. 9, a perspective view of a biostimulator in a deployed state is shown in accordance with an embodiment. The pacing electrode 108, like the fixation element 106, may be movable relative to the fixation guide 120. Accordingly, the fixation element 106 and the pacing electrode 108 may both be movable relative to the hinge 310. Similarly, the pacing electrode 108 can be movable relative to the fixation element 106. For example, the pacing electrode 108 may be extended from the side channel 701 of the fixation guide 120 to cause the pacing tip 608 to extend in the transverse direction (relative to the housing axis 116) into the target tissue. It is understood that, in the deployed state, the pacing axis 114 can parallel the fixation axis 112. Accordingly, the pacing axis 114 can be orthogonal to the housing axis 116. More particularly, the pacing electrode 108 may initially be pointed toward the housing 110 in the aligned substate and then rotated into place to be directed transversely in the misaligned substate. When the biostimulator 100 is in the misaligned substate, the pacing electrode 108 can extend into the target tissue such that the pacing tip 608 is radially outward, relative to the housing axis 116, as compared to the fixation tip 604. The fixation element 106 may therefore anchor the biostimulator 100 to the septal wall 104, and the pacing tip 608 can extend deeper into the target tissue to pace the target anatomy, e.g., a bundle branch. Accordingly, pacing signals can be delivered to the target anatomy through the pacing tip 608 while the housing 110 is anchored along the septal wall 104 out of the way of sensitive tissue structures. Optionally, the fixation element 106 and the pacing electrode 108 can be retracted to allow for repositioning at another location on the septal wall 104.

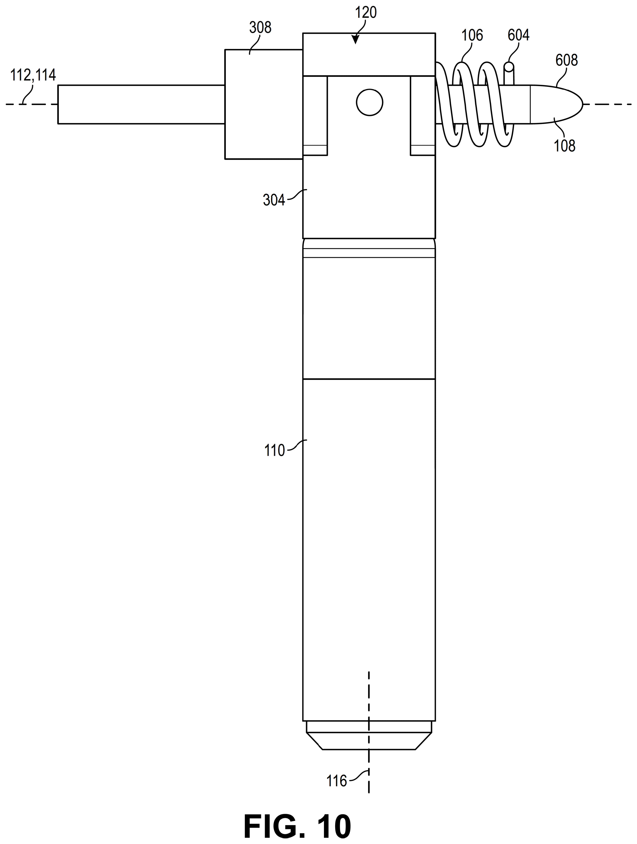

Referring to FIG. 10, a side view of a biostimulator having a fixation guide is shown in accordance with an embodiment. The biostimulator can include coaxial fixation and pacing elements. For example, the pacing electrode 108 and the fixation element 106 may be coaxial. The coaxial relationship between the elements can include the fixation axis 112 being coincident with the pacing axis 114. The coincident axes may extend through the fixation guide 120. For example, the axes may correspond with a central axis of the guide passage 312 of the proximal guide portion 308.

The fixation axis 112 and the pacing axis 114 are shown orthogonal to the housing axis 116 in FIG. 10. Furthermore, the biostimulator 100 is shown in the deployed state and the misaligned substate. More particularly, the biostimulator 100 is shown with the fixation element 106 and the pacing electrode 108 extending out of the guide passage 312. The pacing tip 608 can be radially outward of the fixation tip 604 relative to the housing axis 116. Accordingly, the fixation element 106 can anchor the biostimulator 100 to the septal wall 104 and the pacing electrode 108 can extend into the target tissue to deliver pacing signals to the target anatomy.

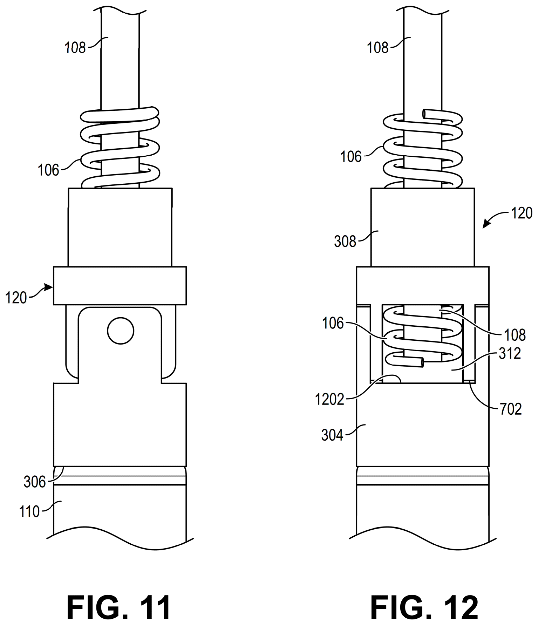

Referring to FIG. 11, a side view of a biostimulator in an undeployed state is shown in accordance with an embodiment. The hinge 310 is shown in the aligned substate. As described above, the fixation element 106 and the pacing electrode 108 may be directed in the distal direction toward the housing 110. The fixation element 106 and the pacing electrode 108 can be in the coaxial relationship, and housed within the fixation guide 120 proximal to the proximal housing end 306 on which the fixation guide 120 is mounted.

Distal ends of the fixation element 106 and the pacing electrode 108 may be positioned proximal to the fixation guide 120 in the aligned substate. The distal ends may be shaped to engage corresponding features of the biostimulator transport system 202. For example, the biostimulator transport system 202 can include a coaxial shaft system. An inner shaft can engage the proximal end of the pacing electrode 108, and an outer shaft can engage the proximal end of the fixation element 106. The biostimulator transport system 202 can include a retractable sleeve, e.g., protective sleeve 208, to surround the portions of the fixation guide 120 during delivery. The retractable sleeve can hold the hinge 310 in place during delivery. To transition the fixation guide 120 from the aligned substate to the misaligned substate, the retractable sleeve can be retracted proximally to expose the fixation guide 120. When exposed, the hinge mechanism can naturally actuate under the weight of the housing 110 to move the biostimulator 100 from the aligned substate to the misaligned substate. The inner shaft can drive the pacing electrode 108 and the outer shaft can drive the fixation element 106. Accordingly, the biostimulator transport system 202 can implant the biostimulator 100 at the septal wall 104.

Referring to FIG. 12, a front view of a biostimulator in an undeployed state is shown in accordance with an embodiment. The front view reveals the fixation element 106 and the pacing electrode 108 positioned within the guide passage 312 of the fixation guide 120. When the fixation element 106 and the pacing electrode 108 are coaxially arranged, both may be located within the guide passage 312. More particularly, the pacing electrode 108 may be in a same passage as the fixation element 106, rather than the side channel 701 as described above. The tips of the fixation element 106 and the pacing electrode 108 can be distal to the distal face 702 of the proximal guide portion 308 and a proximal face 1202 of the distal guide portion 304.

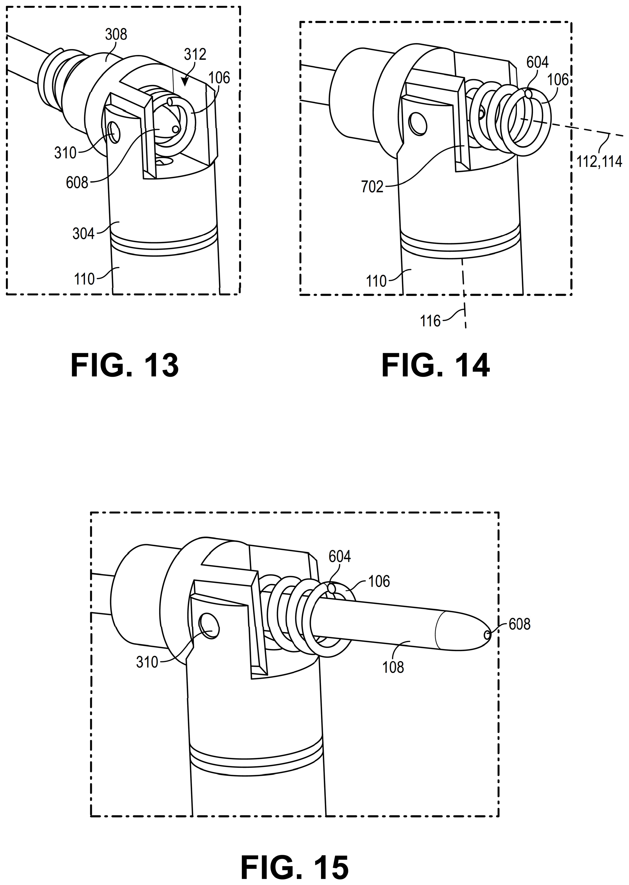

Referring to FIG. 13, a perspective view of a biostimulator having an actuated fixation guide is shown in accordance with an embodiment. In the misaligned substate, the fixation element 106 and the fixation tip 604 can remain within the guide passage 312. For example, when the fixation guide 120 is actuated at the hinge 310 to move the proximal guide portion 308 relative to the distal guide portion 304, the fixation element 106 can remain in place within the guide passage 312. Similarly, the pacing tip 608 can remain within the guide passage 312, coaxial with the fixation element 106.

Referring to FIG. 14, a perspective view of a biostimulator in a deployed state in accordance with an embodiment. When the distal face 702 of the proximal guide portion 308 is pressed against the septal tissue, the fixation element 106 may be advanced to secure the biostimulator 100 to the septal wall 104. As described above, the fixation element 106 can be advanced by rotating the fixation element 106 relative to the proximal guide portion 308. Such rotation threads the fixation element 106 along the protrusion 606 and causes the fixation tip 604 to move out of the guide passage 312. For example, the fixation tip 604 can penetrate tissue and the fixation helix can screw into the target tissue to secure the biostimulator 100 to the septal wall 104. Accordingly, in the deployed state, the fixation tip 604 of the biostimulator 100 can extend out of the guide passage 312.

In the deployed state, when the fixation tip 604 extends out of the guide passage 312, the fixation axis 112 can be oblique and/or orthogonal to the housing axis 116. Such obliqueness and/or orthogonality allows the fixation element 106 to be directed into the septal tissue along which the housing 110 lies parallel against.

Referring to FIG. 15, a perspective view of a biostimulator in a deployed state is shown in accordance with an embodiment. The pacing electrode 108, like the fixation element 106, may be movable relative to the fixation guide 120. Accordingly, the fixation element 106 and the pacing electrode 108 may both be movable relative to the hinge 310. Similarly, the pacing electrode 108 can be movable relative to the fixation element 106. For example, the pacing electrode 108 may be extended from the guide passage 312 by pressing or spinning the electrode to cause it to plunge into the target tissue. Optionally, the fixation element 106 and the pacing electrode 108 can be retracted to allow for repositioning at another location on the septal wall 104.

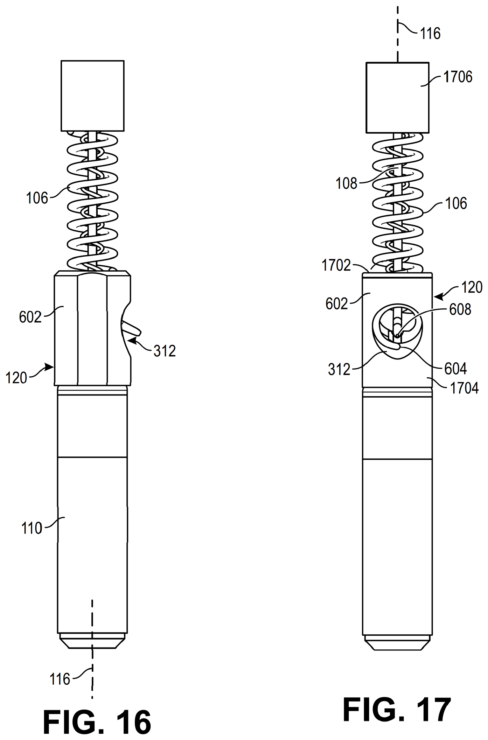

Referring to FIG. 16, a side view of a biostimulator in an undeployed state is shown in accordance with an embodiment. The biostimulator 100 may include a fixation guide 120 without a hinge. The fixation guide 120 can be mounted on the housing 110, and can include the guide passage 312 extending through the guide body 602. The guide passage 312 can guide the fixation helix toward the target tissue. For example, as described below, at least one port of the guide passage 312 can be directed laterally outward in the transverse direction relative to the housing axis 116. Accordingly, fixation element 106 can move through the guide passage 312 to be directed transversely, e.g., in a perpendicular approach through the port, to the septal wall 104.

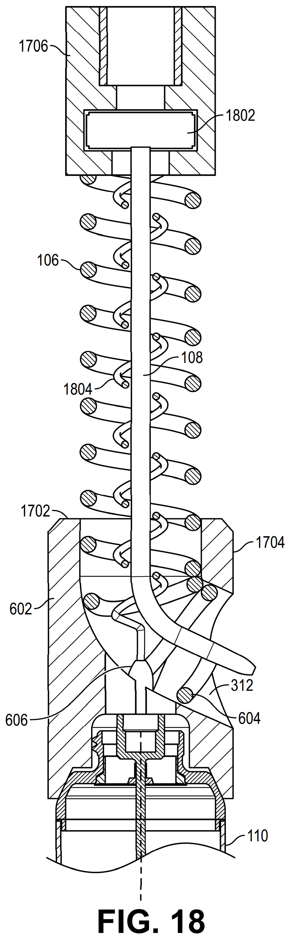

Referring to FIG. 17, a front view of a biostimulator in an undeployed state is shown in accordance with an embodiment. The fixation tip 604 can be positioned within the guide passage 312 when the biostimulator 100 is in the undeployed state. Similarly, the pacing tip 608 can be positioned within the guide passage 312. As shown in the cross-sectional views of FIGS. 18-20, the guide passage 312 can extend through the guide body 602 of the fixation guide 120 from a proximal guide end 1702 to a lateral guide wall 1704 of the fixation guide 120. The guide passage 312 can have ports at those locations. Accordingly, the fixation element 106 and the pacing electrode 108 can be driven downward through the ports and the guide passage 312 to be directed laterally outward into the target tissue.

The fixation element 106 and the pacing electrode 108 can extend proximal to the fixation guide 120. For example, the pacing electrode 108 can extend longitudinally along the housing axis 116, and the pacing electrode 108 can helically extend about the housing axis 116. The pacing electrode 108 and the fixation element 106 can connect to a drive head 1706 located proximal to the housing 110. In an undeployed state, the fixation element 106 and the pacing electrode 108 can be retracted, and a sleeve, e.g., protective sleeve 208, of the biostimulator transport system 202 can surround and store the components. When the biostimulator 100 is positioned within the heart 102 and positioned against the septal wall 104, the sleeve can be retracted and the drive head 1706 can be manipulated to advance the pacing electrode 108 and the fixation element 106 through the guide passage 312 into the target tissue. Such movement into the deployed state is described below.

Referring to FIG. 18, a cross-sectional view of a biostimulator in an undeployed state is shown in accordance with an embodiment. The guide passage 312 can extend along a curvilinear path through the guide body 602. For example, the guide passage 312 can curve from the proximal guide end 1702 facing the proximal direction to lateral guide wall 1704 facing the transverse direction. The shape of the guide passage 312 can redirect the tips of the fixation element 106 and the pacing electrode 108 to direct the components radially outward during tissue engagement.

The fixation element 106 can be moved through the guide passage 312 in a manner similar to the hinged embodiment described above. More particularly, the fixation guide 120 can include the protrusion 606 extending radially inward into the guide passage 312, and the fixation element 106 can be rotated to thread the turns of the helix in a forward or rearward direction. More particularly, spinning the fixation element 106 can move the fixation element 106 along a central axis of the guide passage 312 and follow the path of the guide passage 312.

The pacing electrode 108 can be longitudinally fixed and rotationally independent relative to the fixation element 106. For example, the drive head 1706 can connect to both the fixation element 106 and the pacing electrode 108, and movement of the drive head 1706 can cause movement of both components. The fixation element 106 may be rotationally fixed to the drive head 1706 such that rotation of the drive head 1706 results in rotation of the fixation element 106. By contrast, the pacing electrode 108 can have a proximal hub 1802 connected to a linear electrode body. The proximal hub 1802 can be housed within a recess or cavity of the drive head 1706. Rotation of the drive head 1706 may not be transmitted to the pacing electrode 108. More particularly, when the drive head 1706 rotates, the proximal hub 1802 can spin freely within the cavity. An electrical lead 1804 can extend proximally from the housing 110 through the fixation guide 120 into a central lumen of the fixation element 106. The electrical lead 1804 may pass through the protrusion 606 to avoid contact with the turns of the fixation element 106. More particularly, the electrical lead 1804 can pass through the protrusion 606 and emerge into the guide passage 312 radially inward of the helix of the fixation element 106. Accordingly, the helix can be rotated around the electrical lead 1804.

The electrical lead 1804 can connect to the pacing electrode 108 to deliver pacing signals thereto from the pacing circuitry. The electrical lead 1804 can therefore apply a resistive force to prevent rotation of the pacing electrode 108 and the proximal hub 1802 within the drive head 1706. Thus, when the drive head 1706 is rotated, the fixation element 106 can spin around the pacing electrode 108, but the body of the drive head 1706 can push the proximal hub 1802 forward to plunge the pacing electrode 108 into the target tissue. More particularly, the fixation element 106 and the pacing electrode 108 can move relative to each other in a rotational direction, but they may move in concert in the longitudinal direction.

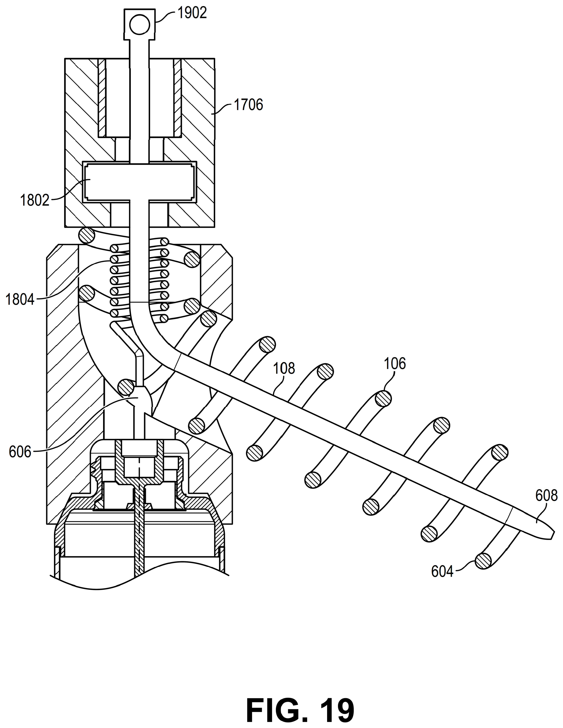

Referring to FIG. 19, a cross-sectional view of a biostimulator in a deployed state is shown in accordance with an embodiment. In the fixed version of the fixation guide 120, the pacing electrode 108 and the fixation element 106 can be driven in and out of the fixation guide 120 in unison. Each spin of the drive head 1706 can rotate the fixation element 106 and also drive the proximal hub 1802 forward or backward. Accordingly, the fixation tip 604 and the pacing tip 608 can be driven into the target tissue and be positioned at a predetermined distance relative to each other. For example, the pacing tip 608 may be located deeper into the target tissue than the fixation tip 604.

The biostimulator 100 may include a tension rod 1902 connected to the proximal hub 1802. The tension rod 1902 may connect to a fiber of the biostimulator transport system 202. For example, the fiber can be looped through a hole in the tension rod 1902. The fiber can be pulled or tensioned to maintain back pressure of the drive head 1706. More particularly, a socket can engage the drive head 1706, and the tension rod 1902 can be manipulated to ensure that the drive head 1706 is securely fit against the socket for effective torque transmission.

When the biostimulator 100 is moved from the undeployed state shown in FIG. 18 to the deployed state shown in FIG. 19, the fixation and pacing components can be driven forward, and the electrical lead 1804 can be compressed. For example, the electrical lead 1804 can spiral around the pacing electrode 108, and the spiral may be stretched in the undeployed state and compressed in the deployed state.

An angle of the guide passage 312 can allow for engagement without requiring significant perpendicular pressure against the septal wall 104. For example, when the biostimulator 100 is delivered to the septal wall 104 with the housing 110 laying along the septal wall 104, the lateral guide wall 1704 of the fixation guide 120 can be apposed to the septal wall 104. The guide passage 312, which curves such that an exit of the passage is facing laterally outward, can guide the pacing electrode 108 in the transverse direction. Accordingly, the pacing electrode 108 and the fixation element 106 can be advanced into the target tissue to perform pacing of the bundle branch.

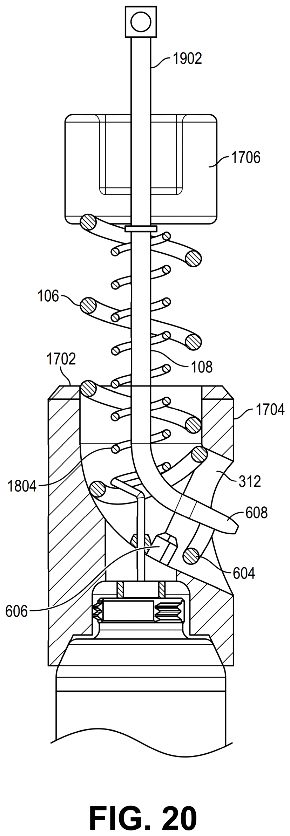

Referring to FIG. 20, a cross-sectional view of a biostimulator in an undeployed state is shown in accordance with an embodiment. The biostimulator 100 can include an independent version of the fixation guide 120 without the hinge 310. In the independent version, the fixation helix and the pacing electrode 108 can be driven separately. Like the fixed version, the independent version can include the guide passage 312 extending downward and laterally within the guide body 602 between the proximal guide end 1702 and the lateral guide wall 1704. The protrusion 606, electrical lead 1804, fixation element 106, and pacing electrode 108 can also have structures similar to those described above. The connection between the fixation element 106, the pacing electrode 108, and the drive head 1706, however, may be different than the fixed version.

In an embodiment, the drive head 1706 is fixed to the fixation element 106. Rotation of the drive head 1706 may therefore drive fixation element 106 forward through the guide passage 312. The pacing electrode 108, however, may not be fixed to or constrained by the drive head 1706. The pacing electrode 108 may be freely rotatable and translatable relative to the drive head 1706. The tension rod 1902 can extend proximally from and/or be integral with the pacing electrode 108. The tension rod 1902 can be pulled or pushed to advance the pacing electrode 108 through the guide passage 312. Such movement may be independent of the fixation element 106 movement.

The independent movement of the fixation element 106 and the pacing electrode 108 allow the components to be delivered into the target tissue to different depths. The drive head 1706 can be rotated to spin the fixation element 106 and screw it forward into the target tissue. The pacing electrode 108 can be pushed to drive the pacing tip 608 into the target tissue. Accordingly, the pacing tip 608 can be driven deeper into the septal wall 104 to engage a bundle branch, while the fixation tip 604 may be located shallower, at the depth required to secure the biostimulator 100 to the septal wall 104. A distance between the tips can be varied based on a clinical situation. The independent electrode may reduce trauma to the septal wall 104 because the fixation element 106 may not be required to advance as deeply as the fixed version.

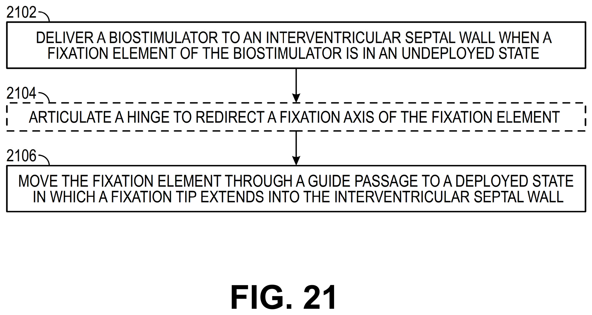

Referring to FIG. 21, a flowchart of a method of implanting a biostimulator for pacing is shown in accordance with an embodiment. At operation 2102, the biostimulator 100 is delivered to the septal wall 104. Delivery of the biostimulator can occur when the fixation element 106 is in the undeployed state, e.g., with the fixation tip 604 housed within the guide passage 312.

During the implantation procedure, the biostimulator transport system 202 can carry the biostimulator 100 into the target heart chamber. When implantation is to be within the right ventricle, the biostimulator transport system 202 can be tracked through the inferior vena cava into the right atrium and across the tricuspid valve into the right ventricle. The distal end of the biostimulator transport system 202 can be steered toward a desired location of the septal wall 104. For example, the target area may be in an upper region of the interventricular septal wall 104.

At operation 2104, optionally, the hinge 310 can be articulated or otherwise actuated to redirect the fixation axis 112 of the fixation element 106. For example, the hinge 310 can be moved from the aligned substate to the misaligned substate to cause the fixation element 106 to be directed in the transverse direction toward the target tissue. More particularly, articulating the hinge 310 can redirect the fixation axis 112 of the fixation element 106 from being parallel to the housing axis 116, in the undeployed state, to being oblique and/or orthogonal to the housing axis, in the deployed state. In the aligned substate, the fixation element 106 can extend downward toward the housing 110 and in the misaligned substate the fixation element 106 can be directed transversely away from the housing axis 116.

At operation 2106, the fixation element 106 is moved through the guide passage 312 to the deployed state. The fixation tip 604 can extend into, e.g., be screwed into, the interventricular septal wall 104. More particularly, the fixation element 106 can be spun through the guide passage 312 to advance the fixation tip 604 into the target tissue. The pacing electrode 108 may be affixed to the interventricular septum. When the fixation element 106 is attached to the tissue, the housing 110 can be secured relative to the septal wall 104, away from sensitive heart structures.

The pacing electrode 108 can be moved through the guide passage 312. Similar to the fixation element 106, the pacing electrode 108 can be spun or rotated to advance forward. Alternatively, the pacing electrode 108 can be pushed or plunged into the tissue. Accordingly, the pacing tip 608 can be located at the target anatomy, e.g., the bundle branch. The target bundle branch can be paced while the housing 110 is optimally placed for stability and reduced risk of tissue trauma.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a housing (110) having an electronics compartment (302) containing pacing circuitry;
a fixation guide (120) mounted on the housing (110), wherein the fixation guide (120) includes a protrusion (606) extending into a guide passage (312); and
a helical fixation element (106) in the guide passage (312), wherein the protrusion (606) is arranged to cause the helical fixation element (106) to axially advance from an undeployed state to a deployed state in response to rotation of the helical fixation element (106) relative to the fixation guide (120).

2. The biostimulator of claim 1, wherein
in the undeployed state a fixation tip (604) of the helical fixation element (106) is within the guide passage (312);
in the deployed state a fixation tip (604) of the helical fixation element (106) extends out of the guide passage (312).

3. The biostimulator of claim 1 or 2, wherein the protrusion (660) acts as a mechanical thread, and wherein turns of the helical fixation element (106) slide over the mechanical thread in response to the rotation.

4. The biostimulator of any one of claims 1 to 3, wherein in the undeployed state a fixation axis (112) of the helical fixation element (106) is parallel to a housing axis (116) of the housing (110).

5. The biostimulator of any one of claims 1 to 4, wherein in the deployed state a fixation axis (112) of the helical fixation element (106) is offset from, preferably orthogonal to, a housing axis (116) of the housing (110).

6. The biostimulator of any one of claims 1 to 5, wherein the fixation guide (120) includes an opening through which a fixation tip (604) of the helical fixation element (106) extends in the deployed state.

7. The biostimulator of any one of claims 1 to 6 further comprising a pacing electrode (108) electrically coupled to the pacing circuitry, wherein the pacing electrode (108) is configured to deliver pacing signals to a target anatomy.

8. The biostimulator of claim 7, wherein the fixation guide (120) includes a side channel (701) separate from and radially offset from the guide passage (312), and wherein the pacing electrode (108) extends through the side channel (701).

9. The biostimulator of any one of claims 1 to 8, wherein the fixation guide (120) includes a hinge (310) movably connecting a distal guide portion (304) mounted on the housing (110) to a proximal guide portion (308) containing the guide passage (312).

10. The biostimulator of claim 9, wherein the proximal guide portion (308) includes a distal face (702) arranged to press against septal tissue during deployment of the helical fixation element (106).

11. The biostimulator of any one of claims 1 to 10 further comprising a stop tab (620) connected to a distal end of the helical fixation element (106), wherein the stop tab (620) is configured to contact the (606) to limit advancement of the helical fixation element (106).

12. The biostimulator of any one of claims 1 to 11, wherein the guide passage (312) extends through a guide body (602) of the fixation guide (120) from a proximal guide end (1702) of the fixation guide (120) to a distal guide end of the fixation guide (120).

13. The biostimulator of any one of claims 1 to 12 further comprising a pacing electrode (108) electrically coupled to the pacing circuitry, wherein the pacing electrode (108) extends along a pacing axis (114), and wherein the pacing electrode (108) is coaxial with the helical fixation element (106).

14. A biostimulator system (200), comprising:
a biostimulator transport system (202); and
the biostimulator (100) of any one of claims 1 to 13 mounted on the biostimulator transport system (202).

15. The biostimulator system of claim 14, wherein the biostimulator transport system (202) is configured to retain and rotate the biostimulator (100).
